# EUROPEAN PATENT SPECIFICATION

(11) **EP 0 578 323 B1**
(45) Date of publication and mention of the grant of the patent: **11.03.1998**
(21) Application number: 93201968.0
(22) Date of filing: 06.07.1993
(51) Int. Cl.: C07C 209/60, C08F 8/32

(54) **Process for the preparation of secondary amines and catalyst compositions for use in such process**
Verfahren zur Herstellung von sekundären Aminen und in diesem Verfahren zu verwendenden Katalysator
Procédé pour la préparation d'amines secondaires et catalyseur utilisé dans ce procédé

(30) Priority: 08.07.1992 EP 92306266
(43) Date of publication of application: 12.01.1994
(73) Proprietor: SHELL INTERNATIONALE RESEARCH MAATSCHAPPIJ B.V., 2596 HR Den Haag (NL)
(72) Inventor: De Boer, Henricus Jacobus Robert, NL-1031 CM Amsterdam 3BD (NL); Savage, Paul David, Sittingbourne, Kent ME10 1XH (GB)

(56) References cited:
- EP-A- 0 444 481
- FR-A- 2 476 655
- GB-A- 2 136 438
- US-A- 4 178 312
- US-A- 4 189 448

## Description

The present invention relates to processes for the preparation of secondary amines and to catalyst compositions suitable for use in the preparation of secondary amines.

Aminomethylation processes in which ammonia or a primary amine is reacted with carbon monoxide, hydrogen and an olefinic compound in the presence of a transition metal catalyst such as a ruthenium and/or rhodium catalyst to produce a secondary amine are well known in the art, e.g. as described in US Patents Nos. 4,503,217, 4,526,936 and 4,832,702, and European Patent Application Publication No. 457,386.

The overall aminomethylation process can be formally divided into three reactions. The first is hydroformylation leading to the formation of aldehyde or metal acyl species followed by condensation, resulting in the intermediate formation of a Schiff's base or enamine, and subsequent hydrogenation of the C=N or C=C-N bond respectively to produce the desired end product amine (J. Org. Chem., 1982, 47, No. 3, pp. 445-447).

The transition metal catalysts that have generally been used in the aforementioned aminomethylation processes are homogeneous catalysts which, being soluble in the reaction medium, are difficult to recover for subsequent use. Catalyst recovery, however, is especially important in the case of rhodium catalysts in view of the high cost of rhodium metal.

Heterogeneous catalysts which, on the other hand, are easily recoverable from the reaction medium are known from US Patents Nos. 3,636,159, 3,652,676, 4,178,314 and 4,189,448. The catalysts described in these patents are said to be useful in hydroformylation reactions i.e. in catalysing the conversion of olefins to aldehydes and, optionally, in hydrogenation reactions, e.g. in catalysing the conversion of aldehydes to alcohols.

More particularly, US Patent No. 3,636,159 discloses heterogeneous hydroformylation catalysts comprising a solid polymer of a pyridine having associated therewith a metal of the group cobalt, rhodium, ruthenium, platinum and palladium. The only catalysts described in the Examples are cobalt-containing catalysts.

US Patent No. 3,652,676 discloses heterogeneous hydroformylation catalysts comprising a solid polymer formed by polymerising a pyridine and a styrene in the presence of a polyvinylaromatic compound, having associated therewith a metal of the group cobalt, rhodium, ruthenium, platinum and palladium. The catalysts described in the Examples are all cobalt-containing catalysts.

In column 2, lines 1 to 7 of US Patent No. 3,636,159 and in column 2, lines 4 to 10 of US Patent No. 3,652,676 it is stated "The materials which can be so employed are cobalt, rhodium, ruthenium, platinum and/or palladium, which are in such a form as to constitute a metal carbonyl under the reaction conditions. In general, cobalt is the preferred metal and advantageously can be in the form of cobalt hydrocarbonyl or dicobalt octacarbonyl.".

There is no disclosure in either of these two US Patents of any catalysts containing metal compounds other than carbonyls or naphthenates per se.

US Patent No. 4,178,314 discloses heterogeneous hydroformylation and hydrogenation catalysts having the general structure Ⓟ - MCl₃ where M is rhodium or iridium and Ⓟ is a heterocyclic nitrogen-containing polymer. It can be seen from the Examples, particularly Examples 7 to 9, of US Patent No. 4,178,314 that the catalysts disclosed do not facilitate simultaneous hydroformylation and hydrogenation of an olefinic feedstock, so that alcohols can only be obtained from the olefinic feedstock via a two-step process involving a first hydroformylation step and a second hydrogenation step.

US-A-4 189 448 discloses a heterogeneous hydroformylation and hydrogenation catalyst having the general structure 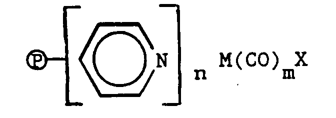 in which n is from 1 to about 3, m is from 1 to about 2, M is rhodium or iridium, X is chlorine, bromine, iodine or hydrogen, and P is a heterocyclic nitrogen-containing polymer. These catalysts are said to allow a simple, two-step, one reactor catalyst synthesis of alcohols from olefins.

French Patent Application Publication No. 2,476,655 discloses a one-step process for forming polymeric polyamines in the presence of a catalytic amount of a rhodium compound, in an inert solvent such as the N-heterocyclic compound N-methyl pyrrolidine.

It has now surprisingly been found that secondary amines can be obtained with a high conversion and selectivity in a one-step process using a particular heterogeneous rhodium catalyst.

Therefore, in accordance with the present invention there is provided a process for the preparation of a secondary amine which comprises reacting a primary amine with carbon monoxide, hydrogen and an olefinic compound in the presence of a catalyst composition comprising a source of cationic rhodium, a source of an anion other than a halide, and, as support, a polymer of a N-heterocyclic compound.

The primary amine used in the process of the present invention may be a monoamine, for example, an alkylamine, a cycloalkylamine or an arylamine, or a polyamine containing at least one primary amino group.

In this specification, unless otherwise stated, an alkyl group or alkyl moiety in an alkoxy group may be linear or branched and preferably contains up to 20, more preferably up to 10, and especially up to 6 carbon atoms. A cycloalkyl group may contain from 3 to 8, preferably 3 to 6, carbon atoms. An aryl group may be any aromatic hydrocarbon group, especially a phenyl or naphthyl group. An alkaryl group may be any aryl group substituted by at least one alkyl group, e.g. a methylphenyl group.

The alkyl, cycloalkyl or aryl group in an alkylamine, a cycloalkylamine or an arylamine may be substituted with one or more, e.g. up to 6, preferably up to 4, substituent groups.

Examples of substituents include alkoxy, alkyl, cycloalkyl, aryl and alkaryl groups.

Examples of monoamines are methylamine, ethylamine, propylamine, n-butylamine, s-butylamine, t-butylamine, cyclopentylamine, cyclohexylamine and aniline.

The polyamine may consist of carbon, hydrogen and nitrogen atoms or it may additionally contain oxygen and/or sulphur atoms. For example, the polyamine may be a compound of the general formula: 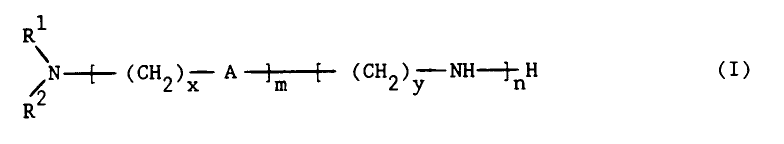 wherein each x is in the range 1 to 3, each y is in the range 1 to 3, each A independently represents -O- or -S-, m is 0, 1 or 2 and n is in the range 1 to 10, and wherein when m is 1, n is 1 and A is -O- or -S- or n is 2 and A is -S-, and when m is 2, n is 1; and each of R¹ and R² independently represents a hydrogen atom or an alkyl group.

In formula I above, it is preferred that each of R¹ and R² independently represents a hydrogen atom or a C₁-C₆ alkyl, especially methyl, group. Particularly preferred polyamines of formula I are those wherein each of R¹ and R² independently represents a hydrogen atom or a methyl group, y is 2 or 3, m is 0 and n is in the range 1 to 6, e.g. 3-dimethylamino-1- propylamine, ethylene diamine, diethylene triamine, triethylene tetramine, tetraethylene pentamine, pentaethylene hexamine and hexaethylene heptamine.

The compounds of formula I above may be prepared by the methods of H.R. Ing and R.H.F. Manske, J. Chem. Soc., 1926, p. 2348 et seq.; D.S. Tarbell, N. Shakespeare, C.J. Claus and J.R. Bunnett, J. Am. Chem. Soc., 1946, 68, p. 1217 et seq.; R.C. O'Gee and H.M. Woodburn, J. Am. Chem. Soc., 1951, 73, p. 1370 et seq.; A.F. McKay, D.L. Garmaise and A. Halsaz, Can. J. Chem., 1956, 34, p. 1567 et seq; Kirk-Othmer Encyclopedia of Chemical Technology, Volume 7 (Wiley Interscience, New York, 3rd edition, 1979) pp 580-602; US Patent No. 2,318,729; S. Gabriel, Berichte, 1891, 24, pp 1110-1121; F.P.J. Dwyer and F. Lions, J. Am. Chem. Soc., 1950, 72, pp 1545-1550; F.P.J. Dwyer, N.S. Gill, E.C. Gyarfas and F. Lions, J. Am. Chem. Soc., 1953, 75, pp 1526-1528; US Patent No. 3,362,996; or by processes analogous thereto.

The olefinic compound used in the process of the present invention contains at least two carbon atoms and may contain 1, 2 or more olefinic bonds. The olefinic compound preferably contains from 2 to 7200, more preferably 2 to 1000, still more preferably 2 to 500, and especially 2 to 100, carbon atoms.

Examples of olefinic compounds include alkenes, cycloalkenes, alkadienes and cycloalkadienes optionally substituted with one or more, e.g. up to 6, preferably up to 4, substituents selected from alkoxy, alkyl, cycloalkyl, aryl and alkaryl groups; and oligomers and polymers thereof (hereinafter referred to as "polyolefins").

An alkene or alkadiene may contain terminal or internal olefinic bonds. Terminal olefinic bonds are preferred.

A particularly preferred olefinic compound is a C₂-C₁₀ alkene, or oligomer or polymer thereof.

Examples of unsubstituted alkenes are ethene, propene, butene, isobutene, the isomeric pentenes or hexenes, 1-octene, diisobutylene, and of substituted alkenes are styrene and methylstyrene. Examples of cycloalkenes are cyclohexene and norbornene.

Examples of alkadienes are 1,5-hexadiene, 1,7-octadiene and 1,9-undecadiene. Examples of cycloalkadienes are norbornadiene and dicyclopentadiene.

The polyolefin may be partially hydrogenated, but it is important that there is some residual olefinic unsaturation in the polyolefin.

The polyolefin has a number average molecular weight (Mₙ) preferably in the range from 200 to 100,000, more preferably from 200 to 5,000, and especially from 500 to 2,500.

Examples of polyolefins are atactic polypropylene which may be conveniently prepared by a process analogous to the process of Comparative Example 1 of EP-A-268 214, or, more generally, to the process decsribed in EP-A-69 951; partially hydrogenated polyisoprene which may be conveniently prepared by a process analogous to Example 1(a) of GB-A-1 575 507 with partial hydrogenation of the resulting polymer in accordance with Example 1(c); polyisobutylene; polyalphaolefins, that is, partially hydrogenated oligomers, primarily trimers, tetramers and pentamers, of alphaolefin monomers containing generally from 6 to 12, more usually 8 to 12, carbon atoms, which may be prepared by a process as outlined in Hydrocarbon Processing, Feb. 1982, page 75 et seq; and, also, hydrocarbons prepared by a plasma process as described in EP-A-346 999.

Polyisobutylenes such as those sold by British Petroleum Company under the Trade Marks "Ultravis", "Hyvis" and "Napvis" are particularly preferred for use in the present invention, e.g. "Ultravis 10", "Ultravis 30", "Hyvis 10", "Hyvis 30", "Hyvis 150" and "Napvis 10" polyisobutylenes having respectively a number average molecular weight (Mₙ) of 970*, 1300*, 1025*, 1320, 2400 and 971*, the Mₙ values marked with an asterisk having been determined by gel permeation chromatography and the unmarked ones by quantitative reaction with ozone.

The process according to the present invention is conveniently carried out at a temperature in the range from 60 to 200°C, preferably from 100 to 200°C, and especially from 125 to 170°C.

The present process is conveniently carried out at a total pressure as determined at ambient temperature (20°C) ranging from 200 to 25,000 kPa, preferably from 2,000 to 20,000 kPa, and especially from 2,000 to 10,000 kPa, in particular 6,000 kPa. It will be appreciated, however, that when the process is operated batchwise at a constant temperature, the pressure will fall during the course of reaction.

A source of cationic rhodium may be any material comprising rhodium which is capable of yielding cationic rhodium species. Examples of suitable sources include compounds of rhodium such as oxides; salts such as nitrates, sulphates, sulphonates (e.g. trifluoromethanesulphonates or p-toluenesulphonates), borates (e.g. tetrafluoroborates such as Rh(CO)₂(BF₄)), phosphates, phosphonates (e.g. benzenephosphonates), carboxylates (e.g. acetates, propionates or butyrates), and acetylacetonates (e.g. Rh(CO)₂(acac) where acac denotes an acetylacetonate anion); and hydrides (e.g. HRh(CO)(Ph₃P)₃).

The source of an anion other than a halide may be a salt or an acid. It may also be a metal complex, for example a rhodium complex. The anion is preferably a nitrate, sulphate, sulphonate, borate, phosphate, phosphonate, carboxylate or acetylacetonate anion. Preferably the source of anion is a complex or salt of rhodium, or an acid.

The N-heterocyclic compound is preferably selected from piperidine, morpholine, piperazine, pyridine, bipyridine, pyrrolidone, pyrrole, benzopyrrole, quinoline, indole, imidazole, carbazole, phenanthroline, each of which is alkenyl-, preferably vinyl- substituted, and 4-methyl-4'-vinyl-2,2'-bipyridine.

More preferably, the N-heterocyclic compound is selected from 2-vinylpyridine, 3-vinylpyridine, 4-vinylpyridine and 4-methyl-4'-vinyl-2,2ʼ-bipyridine.

The polymer of the N-heterocyclic compound may be a homopolymer or a copolymer. Copolymers are preferred.

Comonomers useful in the preparation of the copolymers include N-heterocyclic compounds such as those described above; C₁-C₂₀-, preferably C₁-C₁₀-alkyl(meth)acrylates, especially methyl acrylate; C₂-C₂₀-, preferably C₂-C₁₀-alkenyl(di)- (meth)acrylates, especially ethene diacrylate; and divinylbenzene. Divinylbenzene is a particularly preferred comonomer.

The preferred copolymers are 2-vinylpyridine/ methyl acrylate/ethene diacrylate, 2-vinylpyridine/ divinylbenzene, 3-vinylpyridine/divinylbenzene, 4-vinylpyridine/divinylbenzene and 4-methyl-4'-vinyl-2,2'-bipyridine/divinylbenzene copolymers.

The preparation of a polymer of an N-heterocyclic compound is known in the art. The polymer may conveniently be prepared by heating the N-heterocyclic monomers, optionally with one or more different comonomers, at a temperature in the range from 50 to 150°C in an organic solvent and in the presence of an initiator such as azoisobutyronitrile, e.g. according to the method of Hjortkjaer, Chen and Heinrich, Applied Catalysis, 1991, 67, pp 269-278.

The number of moles of anion per gram atom of rhodium is preferably at least 0.5, more preferably in the range from 1 to 50, yet more preferably from 1 to 25, and especially from 1 to 10.

The number of moles of polymer support per gram atom of rhodium is preferably in the range from 10⁻⁸ to 10⁻¹, more preferably from 10⁻⁷ to 10⁻³, and especially from 10⁻⁶ to 10⁻⁵.

The number of gram atoms of rhodium used per mole of olefinic compound is not critical. It is preferably in the range from 10⁻⁶ to 10² gram atoms rhodium per mole of olefinic compound.

The CO to H₂ ratio preferably ranges from 1:10 to 10:1, more preferably from 1:5 to 5:1, and is especially 1:2.

The process of the present invention may be carried out in the presence of a solvent. Typical examples of solvents are ethers such as diethyleneglycol dimethylether (diglyme), 1,4-dioxane or tetrahydrofuran; amides such as dimethylformamide or dimethylacetamide; alcohols such as hexanol or tetraethylene glycol; esters such as ethyl acetate; and hydrocarbons such as hexane, cyclohexane, toluene and the xylenes.

The present invention further provides a catalyst composition comprising a source of cationic rhodium, a source of an anion other than a halide or hydride, and, as support, a polymer of a N-heterocyclic compound.

The invention will be further understood from the following illustrative examples.

### Example 1

### Preparation of poly(4-vinylpyridine/divinylbenzene) - [Rh(CO)₂][BF₄] heterogeneous catalyst

The reaction was performed in a glovebox. To a suspension of 10.00g poly(4-vinylpyridine) crosslinked with 2% divinylbenzene (available from the Aldrich Chemical Company Ltd., UK) in 600 ml methanol were added 1.849g (4.75 mmol) [Rh(CO)₂Cl]₂ and the reaction mixture was stirred for 48 hours at ambient temperature (20°C). Subsequently, 1.044g (9.50 mmol) NaBF₄ were added to the reaction mixture and stirring was continued for a further 36 hours. The desired end product, poly(4-vinylpyridine/divinylbenzne)-[Rh(CO)₂][BF₄], a solid, was filtered, washed with methanol and water and then dried under vacuum. The end product was obtained in a 98% yield and was found to contain 7.5% Rh as determined by ICP-MS (calc., 7.7%).

### Example 2

### Preparation of secondary amine

Into a 250 ml "Hastelloy C" (Trade Mark) metal autoclave equipped with a magnetic stirring bar and heating mantle were introduced 77 mmol "Hyvis 10" (Trade Mark) polyisobutylene (ex BP, number average molecular weight (Mₙ) 1025 as determined by gel permeation chromatography), 105 mmol 3-dimethylamino-1-propylamine, 75 ml 1,4-dioxane and 0.3 g of the heterogeneous catalyst obtained in Example 1. The contents of the autoclave were stirred whilst the gascap was evacuated (12 kPa). After the gas-feed line had been purged, the autoclave was pressurised with carbon monoxide gas to a pressure of 2000 kPa. A slight decrease in carbon monoxide pressure was observed due to some of the carbon monoxide gas dissolving in the reaction mixture. The carbon monoxide pressure was therefore readjusted to the desired value of 2000 kPa. The autoclave was further pressurised with hydrogen gas (4000 kPa) following the same procedure as described for carbon monoxide to give a total pressure at ambient temperature (20°C) of 6000 kPa. When the pressure was stable, the contents of the autoclave were heated, with stirring, at 170°C for a period of 24 hours. After cooling, the autoclave was depressurised and the contents were poured into a separation funnel. An upper product-containing phase was separated from a lower solvent-containing phase and the former added to 50 ml hexane. The resulting mixture was then washed three times with 50 ml portions of water. Evaporation of the hexane at 110°C and 0.3 kPa using a rotary evaporator yielded the reaction product which was subsequently analysed for active matter (i.e. polyisobutylene conversion) and basic-N, secondary-N and tertiary-N contents).

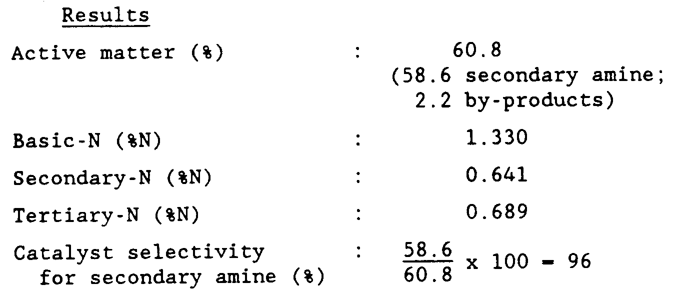

### Example 3

The procedure described in Example 2 was repeated except that 1.0 g of the heterogeneous catalyst obtained in Example 1 was used and the reaction temperature was lowered to 150°C.

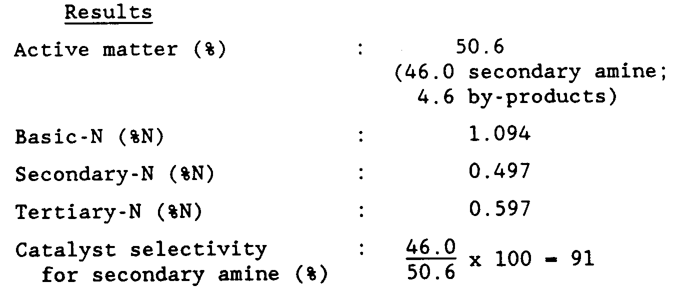

The catalyst was recovered, and recycled twice, with comparable results to the above being obtained on each occasion.

### Example 4

The procedure described in Example 2 was repeated except that 50 mmol 1-octene was substituted for "Hyvis 10" polyisobutylene, 50 ml 1,4-dioxane and 0.5 g of the heterogeneous catalyst of Example 1 were used, and the reaction temperature was maintained at 125°C for a period of 19 hours. Analysis of the reaction product revealed that it comprised 98% secondary amine (i.e. C₉H₁₉-NH-(CH₂)₃-N(CH₃)₂). The catalyst selectivity for the secondary amine was calculated to be 99%.

## Claims

1. A process for the preparation of a secondary amine which comprises reacting a primary amine with carbon monoxide, hydrogen and an olefinic compound in the presence of a catalyst composition comprising a source of cationic rhodium, a source of an anion other than a halide, and, as support, a polymer of a N-heterocyclic compound.

2. A process according to claim 1, wherein the primary amine is a compound of the general formula: 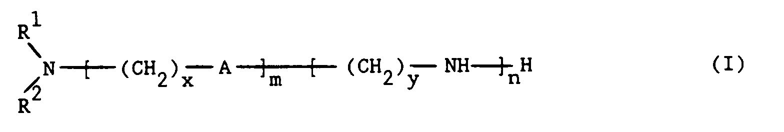 wherein each x is in the range 1 to 3, each y is in the range 1 to 3, each A independently represents -O- or -S-, m is 0, 1 or 2 and n is in the range 1 to 10, and wherein when m is 1, n is 1 and A is -O- or -S- or n is 2 and A is -S-, and when m is 2, n is 1; and each of R¹ and R² independently represents a hydrogen atom or an alkyl group.

3. A process according to claim 1 or claim 2, wherein the olefinic compound is an alkene, cycloalkene, alkadiene or cycloalkadiene, optionally substituted with one or more substituents selected from alkoxy, alkyl, cycloalkyl, aryl and alkaryl groups; or an oligomer or polymer thereof.

4. A process according to any one of claims 1 to 3, wherein the olefinic compound contains from 2 to 100 carbon atoms.

5. A process according to any one of the preceding claims which is carried out at a temperature in the range from 125 to 170°C.

6. A process according to any one of the preceding claims which is carried out at a total pressure in the range from 2,000 to 10,000 kPa.

7. A process according to any one of the preceding claims, wherein the source of cationic rhodium is an oxide, salt or hydride.

8. A process according to any one of the preceding claims, wherein the anion is a nitrate, sulphate, sulphonate, borate, phosphate, phosphonate, carboxylate or acetylacetonate anion.

9. A process according to any one of the preceding claims, wherein the N-heterocyclic compound is selected from piperidine, morpholine, piperazine, pyridine, bipyridine, pyrrolidone, pyrrole, benzopyrrole, quionoline, indole, imidazole, carbazole, phenanthroline, each of which is alkenyl-substituted, and 4-methyl-4'-vinyl-2,2'-bipyridine.

10. A catalyst composition comprising a source of cationic rhodium, a source of an anion other than a halide or hydride, and, as support, a polymer of a N-heterocyclic compound.

## Patentansprüche

1. Verfahren zur Herstellung eines sekundären Amins, welches ein Umsetzen eines primären Amins mit Kohlenmonoxid, Wasserstoff und einer olefinischen Verbindung in Anwesenheit einer Katalysatorzusammensetzung umfaßt, die eine Quelle für kationisches Rhodium, eine Quelle für ein von einem Halogenid unterschiedliches Anion und, als Träger, ein Polymer einer N-heterocyclischen Verbindung umfaßt.

2. Verfahren nach Anspruch 1, worin das primäre Amin eine Verbindung mit der allgemeinen Formel: 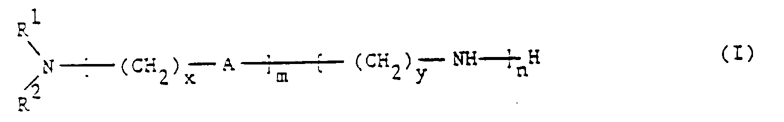 ist, worin jedes x im Bereich von 1 bis 3 liegt, jedes y im Bereich von 1 bis 3 liegt, jedes A unabhängig für -0- oder -S- steht, m den Wert 0, 1 oder 2 hat und n im Bereich 1 bis 10 liegt, und worin dann, wenn m den Wert 1 hat, n für 1 und A für -0- oder -S- steht oder n für 2 und A für -S- steht, und dann, wenn m den Wert 2 hat, n für 1 steht; und jeder der Reste R¹ und R² unabhängig ein Wasserstoffatom oder eine Alkylgruppe darstellt.

3. Verfahren nach Anspruch 1 oder 2, worin die olefinische Verbindung ein Alken, Cycloalken, Alkadien oder Cycloalkadien, gewünschtenfalls substituiert durch einen oder mehrere, unter Alkoxy-, Alkyl-, Cycloalkyl-, Aryl- und Alkarylgruppen ausgewählte Substituenten; oder ein Oligomer oder Polymer hievon ist.

4. Verfahren nach einem der Ansprüche 1 bis 3, worin die olefinische Verbindung 2 bis 100 Kohlenstoffatome enthält.

5. Verfahren nach einem der vorstehenden Ansprüche, das bei einer Temperatur im Bereich von 125 bis 170°C ausgeführt wird.

6. Verfahren nach einem der vorstehenden Ansprüche, das bei einem Gesamtdruck im Bereich von 2000 bis 10.000 kPa ausgeführt wird.

7. Verfahren nach einem der vorstehenden Ansprüche, worin die Quelle für kationisches Rhodium ein Oxid, Salz oder Hydrid ist.

8. Verfahren nach einem der vorstehenden Ansprüche, worin das Anion ein Nitrat-, Sulfat-, Sulfonat-, Borat-, Phosphat-, Phosphonat-, Carboxylat- oder Acetylacetonatanion ist.

9. Verfahren nach einem der vorstehenden Ansprüche, worin die N-heterocyclische Verbindung unter Piperidin, Morpholin, Piperazin, Pyridin, Bipyridin, Pyrrolidon, Pyrrol, Benzopyrrol, Chinolin, Indol, Imidazol, Carbazol, Phenanthrolin, jeweils alkenylsubstituiert, und 4-Methyl-4'-vinyl-2,2'-bipyridin ausgewählt ist.

10. Katalysatorzusammensetzung, umfassend eine Quelle für kationisches Rhodium, eine Quelle für ein von einem Halogenid unterschiedliches Anion oder eine Hydrid und, als Träger, ein Polymer einer N-heterocyclischen Verbindung.

## Revendications

1. Procédé de préparation d'une amine secondaire, caractérisé en ce que l'on fait réagir une amine primaire avec le monoxyde de carbone, d'hydrogène et un composé oléfinique en présence d'une composition catalytique comprenant une source de rhodium cationique, une source d'un anion autre qu'un halogénure et, à titre de support, un polymère d'un composé N-hétérocyclique.

2. Procédé suivant la revendication 1, caractérisé en ce que l'amine primaire est un composé de la formule générale suivante : 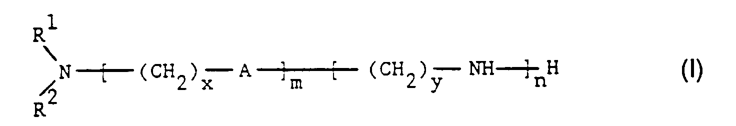 dans laquelle chaque x a une valeur qui varie de 1 à 3, chaque y a une valeur qui varie de 1 à 3, chaque symbole A représente indépendamment -O- ou -S-, m est égal à 0, 1 ou 2 et n a une valeur qui varie de 1 à 10, et dans laquelle, lorsque m est égal à 1, n est égal à 1 et A est -O- ou -S-, ou n est égal à 2 et A est -S- et lorsque m est égal à 2, n est égal à 1; et dans laquelle chacun des symboles R¹ et R² représente indépendamment un atome d'hydrogène ou un radical alkyle.

3. Procédé suivant la revendication 1 ou la revendication 2, caractérisé en ce que le composé oléfinique est un alcène, un cycloalcène, un alcadiène ou un cycloalcadiène, éventuellement substitué par un ou plusieurs substituants choisis parmi les radicaux alcoxy, alkyle, cycloalkyle, aryle et alcaryle; ou un oligomère ou un polymère de ce composé.

4. Procédé suivant l'une quelconque des revendications 1 à 3, caractérisé en ce que le composé oléfinique contient de 2 à 100 atomes de carbone.

5. Procédé suivant l'une quelconque des revendications précédentes, caractérisé en ce qu'on l'entreprend à une température comprise entre 125 et 170°C.

6. Procédé suivant l'une quelconque des revendications précédentes, caractérisé en ce qu'on l'entreprend à une pression totale qui varie de 2000 à 10 000 kPa.

7. Procédé suivant l'une quelconque des revendications précédentes, caractérisé en ce que la source de rhodium cationique est un oxyde, un sel ou un hydrure.

8. Procédé suivant l'une quelconque des revendications précédentes, caractérisé en ce que l'anion est un anion nitrate, sulfate, sulfonate, borate, phosphate, phosphonate, carboxylate ou acétylacétonate.

9. Procédé suivant l'une quelconque des revendications précédentes, caractérisé en ce que l'on choisit le composé N-hétérocyclique parmi la pipéridine, la morpholine, la pipérazine, la pyridine, le bipyridine, la pyrrolidone, le pyrrole, le benzopyrrole, la quinoléine, l'indole, l'imidazole, le carbazole, la phénanthroline, chacune d'entre elles étant alcényl-substituée et la 4-méthyt-4'-vinyl-2,2'-bipyridine.

10. Composition catalytique, caractérisée en ce qu'elle comprend une source de rhodium cationique, une source d'un anion autre qu'un halogénure ou un hydrure et, à titre de support, un polymère d'un composé N-hétérocyclique.
